# EUROPEAN PATENT APPLICATION

(11) **EP 2 995 934 A1**
(43) Date of publication of application: **16.03.2016**
(21) Application number: 14184820.0
(22) Date of filing: 15.09.2014
(51) Int. Cl.: G01N 21/65, G01J 3/44, B82Y 5/00, B82Y 15/00, G01N 33/487, C12Q 1/68, B82Y 35/00, G02B 6/122, C23C 16/455

(54) **Improved nanopore plasmonic analyser**

(71) Applicant: Base4 Innovation Ltd, Cambridge, Cambridgeshire CB3 0FA (GB)
(72) Inventor: The designation of the inventor has not yet been filed
(74) Representative: Lau, Sarah Jane

(57) **Abstract**

An apparatus for investigating a molecule is provided. It includes:
• a chip comprising (a) a substrate perforated with at least one nanopore and (b) at least one pair of nanostructures juxtaposed about the nanopore thereby defining a gap in which an electromagnetic field can be generated by localised surface plasmon resonance;
• first and second chambers arranged either side of the chip and linked by the nanopore(s);
• a source of incident electromagnetic radiation for inducing localised surface plasmon resonance in the nanostructures:
• a controller for causing molecules to pass from the first to the second chambers via the nanopore(s) and through the electromagnetic field and
• a detector for detecting Raman-scattered radiation produced by the molecules as they pass through the electromagnetic field.

The apparatus is characterised in that the gap between the nanostructures in each pair is less than 10 nanometres and that each pair of nanostructures has been prepared by coating a pair of precursor nanostructures that are separated by greater than or equal to 10 nanometres. The apparatus is suitable for sequencing nucleic acids and proteins.

## Description

This invention relates to an improved nanopore plasmonic apparatus for investigating molecules using Raman spectroscopy. It is especially useful for investigating biomolecules for example in the sequencing of nucleic acids such as DNA and RNA.

In our previously published patent application WO 2009/030953 we have disclosed an apparatus for investigating a molecule which comprises a nano-perforated substrate provided with pairs of plasmonic structures of nano dimensions juxtaposed within or adjacent the nanopores. In this apparatus, the gaps between the pairs of plasmonic structures define detection windows comprising an electromagnetic field within which the analyte molecule (optionally labelled) is induced to fluoresce or Raman-scatter photons in characteristic way by interaction with incident light. The photons so generated are then detected remotely, multiplexed and converted into a data-stream whose information content is characteristic of the molecular structure of the analyte itself. This information can then be recovered from the data-stream using computational algorithms embodied in corresponding hardware or software programmed into a microprocessor integral therewith or in a separate computing apparatus attached thereto. In one useful embodiment, the analyte is a nucleic acid and the apparatus is used to determine the sequence of its constituent nucleotide bases by electrophoretically translocating the nucleic acid base-by-base through the nanopores of the substrate into the detection window(s).

US 2005/0084912 discloses another apparatus for examining inter alia the sequence of nucleotide bases in a DNA sample as it translocates through a nanopore. Here, the apparatus suitably comprises a microscope detector consisting of an optical system comprising a nanolens assembly provided with one or more plasmon resonant particles. The optical system is arranged to be moveable and separate from the substrate bearing the nanopore.

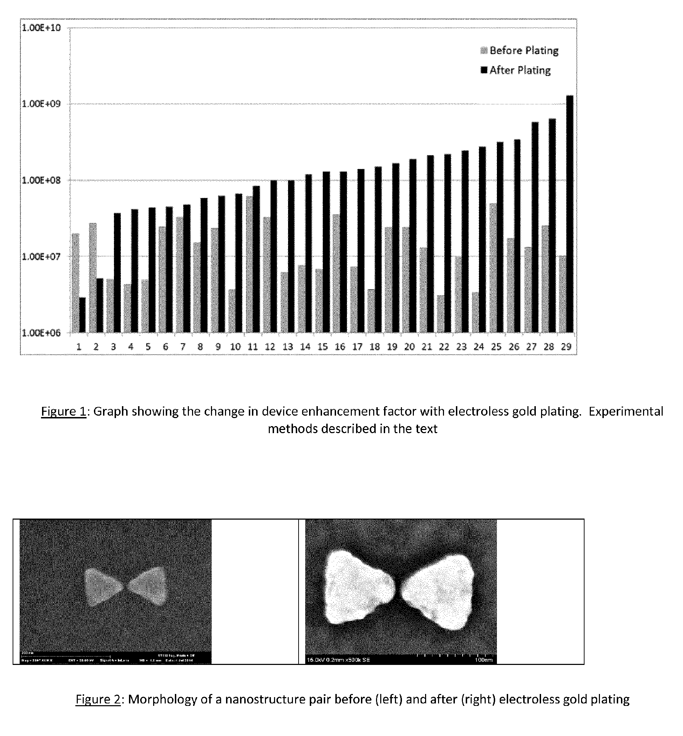

US 2008/0239307 teaches a surface-enhanced Raman-scattering (SERS) method and a corresponding apparatus for sequencing polymeric biomolecules such as DNA, RNA or proteins. In the method disclosed, metallic nanostructures are caused to undergo plasmonic resonance thereby creating an electrical field enhancement near their surface which in turn can improve the Raman-scattering cross-section of any biomolecule located nearby. In Figure 1 of this application an arrangement is shown in which the nanostructures are arranged on the surface of a nanoperforated substrate so that the biomolecule may be caused to translocate both through the nanopore and between the nanostructures.

WO 2011/076951 discloses a similar system for manipulating and analysing biomolecules using nanostructures and associated forces created by plasmon resonance. In one embodiment, it is taught that the system can also comprise a SERS detector.

Finally, JP 2010/0243267 and WO 2012/043028 disclose apparatuses in which biopolymers are caused to translocate through a perforated substrate coated with a thin metal film. Raman-scattering of light from the biopolymer is then analysed using a Raman detector.

A number of factors influence the degree of Raman signal enhancement produced by the plasmonic nanostructures in such devices; key amongst which is the size of the gap between the nanostructures in each pair. Ideally this should be as small as possible, whilst still allowing the analyte molecule to pass between them after exiting the nanopore (greater than approximately 0.5 nanometres). However, consistently manufacturing uniform gaps of less than 10 nanometres can in practice be very difficult using conventional techniques such as e-beam lithography, focused ion beam processing and the like.

We have now overcome this problem by employing coated nanostructures which can be more easily fabricated.

Thus according to the present invention there is provided an apparatus for investigating a molecule including:
- a chip comprising (a) a substrate perforated with at least one nanopore and (b) at least one pair of nanostructures juxtaposed about the nanopore thereby defining a gap in which an electromagnetic field can be generated by localised surface plasmon resonance;
- first and second chambers arranged either side of the chip and linked by the nanopore(s);
- a source of incident electromagnetic radiation for inducing localised surface plasmon resonance in the nanostructures;
- a controller for causing molecules to pass from the first to the second chambers via the nanopore(s) and through the electromagnetic field and
- a detector for detecting Raman-scattered radiation produced by the molecules as they pass through the electromagnetic field;
characterised in that the gap between the nanostructures in each pair is less than 10 nanometres and that each pair of nanostructures has been prepared by coating a pair of precursor nanostructures that are separated by greater than or equal to 10 nanometres.
In an embodiment of the invention the apparatus further comprises a microprocessor or computer for analysing a signal produced by the detector.

The apparatus of the present invention can be used for a number of different duties. For example, in one embodiment it can be used to detect biological pathogens or poisons in drinking water, cooling fluids or air; to detect residual pesticides and pollutants in aqueous extracts taken from soils and foodstuffs and to detect narcotics, explosives or their residua on hard surfaces, clothing or the like. In these duties, the apparatus may be positioned in a guard location with the triggering of an alarm being the intended output.

In another embodiment the apparatus can be used to investigate the nature and disposition of the constituent monomer units of an organic polymer. A preferred duty in this respect is the investigation of the internal structure of biomolecules such as proteins and nucleic acids; especially the determination of the nature of the amino acids in proteins or nucleotides in nucleic acids.

A most preferred duty of all is the sequencing of the nucleotides in natural-occurring or synthetic DNA or RNA. In this duty the apparatus is for example used to sequence polynucleotide fragments greater than 50 nucleotides, preferably greater than 150 nucleotides, most preferably greater than 250 nucleotides long.

The substrate employed in the apparatus of the invention is suitably made of a dielectric material such as glass, silicon or silicon nitride and in one embodiment is a sheet of such material. However the use of a composite sheet for example in which the dielectric material provides the surface(s) bearing the nanostructure(s) is also envisaged. Typically the substrate is 5 to 200 nanometres thick preferably 20 to 200 nanometres. In one embodiment, the substrate comprises a membrane made of silicon, silicon oxide, silicon nitride or materials with equivalent properties. In another, the membrane is mounted on a support made of dielectric support. In yet another, the substrate may include a layer which can function as a mirror for the incident electromagnetic radiation.

The substrate is perforated with one or more nanopores which may be disposed in any arrangement including in a regular array across the whole or a part thereof. Typically, each nanopore has a diameter in the range 0.5 to 100 nanometres, preferably from 0.5 to 50 nanometres most preferably from 0.5 to 10 nanometres. Useful results can be obtained using nanopores whose diameters lie in the range 0.5 to approximately 5 nanometres. In addition to being nanoporous, one or more surfaces of the substrate can be further provided with wells, cavities or channels of nanodimensions.

Turning to the pair(s) of nanostructures, these are juxtaposed opposite each other around the nanopore(s) and are sized and arranged so that localised surface plasmons, as opposed to surface plasmon polaritons, can be induced therein. An advantage of this arrangement is that, by judicious choice of size, the nanostructures can be tuned to produce optimum plasmonic field intensity and density for a given wavelength of the incident electromagnetic radiation. In practice, this means that the each nanostructure should have a maximum dimension of less than 1 micron, preferably in the range 30 to 500 nanometres, most preferably in the range 50 to 250 nanometres. In one embodiment, the pairs of nanostructures are disposed in a regular disposition on a surface of the substrate and about the outlets of the nanopores. In another embodiment, each nanostructure is spaced apart from its pair by less than 9 nanometres or less than 8 nanometres, preferably from 0.5 to 7.5 nanometres, most preferably 0.5 to 5 nanometres in order to induce a strong electromagnetic field which causes enhancement of any Raman-scattering signal obtained from the molecule being investigated.

The nanostructures themselves are typically fabricated from a wide range of metals or dielectric materials coated with metal. Preferred however are those metals which are capable of undergoing plasmon resonance to a significant extent, for example, gold, silver, copper, aluminium, platinum, palladium, molybdenum and chromium and alloys thereof. Most preferably, the metal used will be gold, silver, copper or an alloy thereof. To improve the performance of the apparatus further, the nanostructure may have attached to its surface binding sites which are specifically adapted to capture the particular molecule of interest thereby enhancing the characteristic spectroscopic emissions being sought. Such reactive groups can work by any chemical or physical means; for example when detecting say a signal characteristic of a bacterial pathogen, e.g. the Legionella bacterium, the reactive group can comprise a polynucleotide probe adapted to bind to certain unique base-pair sequences in the bacterium DNA by hybridisation.

It is feature of the apparatus of the present invention that each pair of nanostructures on the substrate has been prepared by coating a pair of precursor nanostructures separated from each other by a greater than or equal to 10 nanometres. Typically this can be achieved by using a method comprising the steps of (1) juxtaposing at least one pair of precursor nanostructures on the substrate about at least one nanopore wherein the precursor nanostructures in the pair are separated by greater than or equal to 10 nanometres and (2) thereafter coating the precursor nanostructures with a coating material selected from a metal capable of exhibiting plasmonic behaviour or a dielectric having a high dielectric index thereby reducing the gap to less than 10 nanometres or less than 9 or less than 8 nanometres; preferably between 0.5 to 7.5 nanometres, most preferably 0.5 to 5 nanometres.

In one embodiment, the coating material is selected from a metal or a dielectric material and in one preferred embodiment it is either gold or titanium dioxide. In another preferred embodiment the precursor nanostructures and the coating material comprises a plasmonic material; preferably the same plasmonic material. Preferably, the coating is made of the same material as the precursor nanostructure, for example gold on gold. In this case the increase in enhancement is expected as the effect is to increase the size of the structures, in turn reducing the gap between them.

When the coating material to be applied is a plasmonic metal such as gold, one suitable method is to coat only the precursor nanostructures using Electroless Metal Plating by a chemical means; for example by depositing the metal onto the precursor nanostructures using a solution of metal ions and a reducing agent such as a borane or borohydride (see for example Schlesinger and Paunovic 'Modern Electroplating', 5th Edition (2010), chapter 21); an ascorbic acid salt (J. Electrochem. Soc., (1995) pp.2250-5) or the cyanide-free system described in US 5935306. Preferably the coating method to be used is self-terminating, in that a finite gap, preferably of the order of less than 2nm is always maintained. In one embodiment, mechanism for self-termination is based on steric effects; i.e. the reactive metal ions or the like are limited in their ability to diffuse into the small gap between the structures.

Alternatively, the coating material to be applied is a dielectric material. In this case, the benefit is not from the closer proximity of the plasmons, rather it is the modification of the refractive index medium in the vicinity of the structures at the frequency of the incident electromagnetic radiation that is responsible for the improved coupling. Ordinarily the devices will be operated in the presence of an aqueous buffer solution carrying the analyte, having a refractive index greater than 1; for example between 1.2 and 1.5. When the gap between the nanostructures is partially occupied by a coating material having such a higher refractive index, then the plasmonic coupling between the nanostructures is stronger and the electromagnetic field intensity increased. In this case, the coating material may be anything with a refractive index higher than that of the buffer solution and typically in the range from 1.5 to 2.0; for example silica (n=1.53 @785nm). Whilst such a coating material may be deposited by chemical vapour deposition, a method particularly suited for depositing it is atomic layer deposition (ALD). ALD is a technique primarily used for depositing metal oxides, and example refractive indices for coating materials of this type are shown in the table below.

| **Material** | **Refractive index at λ=785nm** |
|---|---|
| HfO₂ | 1.89 |
| TiO₂ | 2.52 |
| ZnO | 1.96 |
| Al₂O₃ | 1.65 |

When the coating material to be applied is a dielectric material such as titanium dioxide the technique of Atomic Layer Deposition can be used to treat the whole of the chip so that both the precursor nanostructures and the substrate surface are covered.

In another embodiment, the precursor nanostructures are characterised by having a morphology which in a plane parallel to that of the substrate corresponds to the sector of a circle. Suitably the central angle associated with the sector is in the range 30 to 150 degrees or 30 to 120 degrees; for example 30 to 60 or 60 to 90 degrees. Such precursor nanostructures are hereinafter referred to as 'sector precursor nanostructures'. By the term 'sector of a circle' is meant both a precise geometric sector of a circle and slight deviations therefrom which show superiority over the prior art 'bow-tie' arrangements of nanostructure pairs wherein the morphology in the plane parallel to that of the substrate corresponds to that of a triangular wedge. The precise morphology of the sector precursor nanostructures can for example be determined by scanning electron microscopy. In one embodiment of the invention, each pair of sector precursor nanostructures are enclosed within a separate annulus of plasmonic material of one of the types described above. In another this annulus may be comprised of a pair of half-annuli; e.g. annuli separated along an axis perpendicular to that defined by the foci of the sectors. This arrangement of half-annuli has the additional advantage that they are easier to fabricate; in particular they do not suffer fracturing problems when the 'lift-off' processing method described in the next paragraph is employed. The annuli or half-annuli can themselves function as precursor and therefore also be subsequently coated.

The precursor nanostructures are suitably prepared by first completely coating the substrate with a film of the coating material e.g. a gold film, then masking up the coated product and finally etching away the remaining exposed metal and substrate with a chemical or an ion beam to leave discrete precursor nanostructure pairs arranged about the nanopores. Methods for carrying out such preparations are well known in the art. In one embodiment, the precursor nanostructures may be prepared using lift-off processing. This involves patterning a resist film of a polymer such as poly methyl methacrylate on the substrate by exposure to electron beam irradiation, followed by developing and then metallisation. The resist film is then dissolved away, removing the metal everywhere except where the film was developed.

In a typical working form, the apparatus comprises one or more nanoperforated substrates and their associated nanostructures arranged in a vessel so as to divide the latter into first and second chambers for respectively providing and receiving molecules of the analyte. The apparatus is also provided with a controller for causing molecules of the analyte to translocate from the first chamber to the second chamber via the nanopore(s) and through the electromagnetic field. Typically this controller works by establishing a gradient between the chambers along which the analyte molecule is then induced to pass. Such gradients may include for example those generated by variations in osmotic pressure, or magnetic field or by using chemical reactions to generate a thermodynamic or entropic gradient. However, in one preferred embodiment the gradient is created by an electric field produced by applying a potential difference between the contents of the first and second chambers. The analyte molecule is then caused to translocate by electrophoresis, or by dielectrophoresis in the case of uncharged, polarisable molecules. When the analyte molecule comprises a protein or a nucleic acid the controller preferably employs electrophoresis. In one embodiment, the direction of the potential difference can be reversed; for example to remove molecules of the analyte from the substrate after they have been detected so that the substrate can be cleaned. In another embodiment, the means for establishing this potential difference and the detector may be connected via a feedback loop so that, once the detector detects an expected Raman signal, the direction of the electric field can be varied with time to hold the analyte molecule in the electromagnetic field, fine tune its velocity therethrough or allow resequencing.

The potential difference referred to above can be achieved by locating a first electrode in the first chamber, locating a second electrode, of opposite polarity, in the second chamber and then applying a voltage therebetween. When the analyte is a nucleic acid, the device may additionally include a means to control the speed and orientation of translocation of the analyte through the nanopore, this may be effected for example by means of changes to the solution conditions, gels, modifications to the pore or optical, magnetic or electrical traps.

The remainder of the apparatus comprises a high-intensity source of incident electromagnetic radiation, for example a laser or the like, a detector for detecting the characteristic Raman-scattering and any necessary ancillary optics and electrical circuitry. Either or both of the source of incident electromagnetic radiation and the detector can be continuously associated with some or all of the nanostructure pairs. Alternatively, the nanostructure pairs and the gap between them can be scanned using for example a movable microscope or raster arrangement. The Raman-scattered radiation detected by the detector can in principle be any scattering which is uniquely characteristic of one or more vibrational modes of the analyte and can be derived from single- or multi-photon events.

The nature of the detector employed in the apparatus is not critical and can suitably comprise for example a photodetector, a single photon avalanche diode, an electron-multiplying charge-coupled apparatus or a complementary metal oxide semiconductor apparatus. The detector can additionally be attached to a microprocessor, PC and/or an alarm to process the signal derived therefrom.

Whilst it is envisaged that the whole apparatus can be of unitary design it can also advantageously consist of two components for example a permanent housing in which the source of incident electromagnetic radiation, the detector and optics are located and a disposable cartridge comprising a vessel containing the chambers, chip(s) and nanostructures linked together by microfluidic pathways. In this case the vessel then can be made of for example glass, silicon or plastic. If the apparatus is of unitary design and is used as a chemical sensor e.g. for guard duties, it may be provided with a wireless transmitter to send the signal from the detector back to a central location. The apparatus can then be positioned remotely in which case it may employ a stand-alone power source such as battery if so desired.

The invention is now illustrated by the following Example.

### Example

The enhancement factor for 29 gold nanostructure pairs (bow-tie arrangement) on a silicon nitride substrate was calculated by comparing the degree of enhancement of a reference Raman-scattering signal from sodium cacodylate (605cm⁻¹) against a reference substrate coated with a gold film. In each case, measurements were carried out before and after gold precursor nanostructure were coated with gold by using an electroless plating bath comprising sodium gold sulphite and sodium thiosulphate at 30°C. The target gold film thickness was 3nm, resulting in a decrease in the gap of approximately 6nm.

The results are shown in Figures 1 and 2 which respectively show the degree of enhancement obtained before and after the coating material has been applied by plating and the change in morphology of an example nanostructure pair. Figure 1 shows that plating led to considerable field and therefore signal enhancement in all but two of the 29 instances. In the 27 instances where enhancement occurred it was up to two orders of magnitudes in extent.

## Claims

1. An apparatus for investigating a molecule including:
• a chip comprising (a) a substrate perforated with at least one nanopore and (b) at least one pair of nanostructures juxtaposed about the nanopore thereby defining a gap in which an electromagnetic field can be generated by localised surface plasmon resonance;
• first and second chambers arranged either side of the chip and linked by the nanopore(s);
• a source of incident electromagnetic radiation for inducing localised surface plasmon resonance in the nanostructures:
• a controller for causing molecules to pass from the first to the second chambers via the nanopore(s) and through the electromagnetic field and
• a detector for detecting Raman-scattered radiation produced by the molecules as they pass through the electromagnetic field
**characterised in that** the gap between the nanostructures in each pair is less than 10 nanometres and that each pair of nanostructure has been prepared by coating a pair of precursor nanostructures that are separated by greater than or equal to 10 nanometres.

2. An apparatus as claimed in claim 1 **characterised in that** the gap between the coated nanostructures is from 0.5 to 7.5 nanometres.

3. An apparatus as claimed in either of claim 1 or claim 2 **characterised in that** coating of the precursor nanostructures has been carried out by either Electroless Metal Plating or Atomic Layer Deposition.

4. An apparatus as claimed in any one of claims 1 to 3 **characterised in that** the coating material comprises a metal oxide having a refractive index in the range from 1.5 to 2.0 at the frequency of the incident electromagnetic radiation.

5. An apparatus as claimed in claim 4 **characterised in that** the precursor nanostructures are made of gold and the coating material is selected from hafnium dioxide, titanium dioxide, zinc oxide, silicon dioxide and alumina.

6. An apparatus as claimed in any one of claims 1 to 3 **characterised in that** the both the precursor nanostructures and the coating material are made of gold.

7. An apparatus as claimed in any of the preceding claims **characterised in that** the nanostructures are juxtaposed opposite each other around the nanopore and have morphology in a plane parallel to that of the substrate comprising sectors of a circle having a central angle of from 30 to 150 degrees.

8. An apparatus as claimed in claim 6 **characterised in that** the central angle is from 45 to 90 degrees.

9. An apparatus as claimed in any of the preceding claims **characterised in that** each pair of nanostructures are surrounded by a separate annulus of material also capable of undergoing plasmon resonance.

10. An apparatus as claimed in claim 9 **characterised in that** the annulus is comprised of a pair of half-annuli separated along an axis perpendicular to that defined by the foci of the sectors.

11. An apparatus as claimed in any of the preceding claims **characterised in that** the substrate further comprises a mirror layer.

12. An apparatus as claimed in any of the preceding claims **characterised in that** each nanostructure has a maximum dimension of from 50 to 250 nanometres.

13. An apparatus as claimed in claim 1 **characterised in that** the controller comprises a pair of electrodes respectively located in the first and second chambers.

14. A method for preparing a nanoperforated plasmonic substrate **characterised by** comprising the steps of (1) juxtaposing at least one pair of precursor nanostructures on the substrate about at least one nanopore wherein the precursor nanostructures in the pair are separated by a gap of greater than or equal to 10 nanometres and (2) thereafter coating the precursor nanostructures with a coating material selected from a metal capable of exhibiting plasmonic behaviour or a dielectric having a high dielectric index thereby reducing the gap to less than 10 nanometres.

15. A method as claimed in claim 14 **characterised in that** the coating is carried out using either Electroless Metal Plating or Atomic Layer Deposition.
